(19) Europäisches Patentamt
European Patent Office
Office européen des brevets

(11) Veröffentlichungsnummer : **0 066 195**
**B1**

(12) # EUROPÄISCHE PATENTSCHRIFT

(45) Veröffentlichungstag der Patentschrift : **28.08.85**

(21) Anmeldenummer : **82104391.6**

(22) Anmeldetag : **19.05.82**

(51) Int. Cl.[4] : **C 07 D213/50**, C 07 D207/32, C 07 D239/26, C 07 D239/34, C 07 D231/12, A 01 N 43/34, A 01 N 43/48

(54) **Cyclohexan-1,3-dionderivate, Verfahren zu ihrer Herstellung und ihre Verwendung zur Bekämpfung von unerwünschtem Pflanzenwuchs.**

(30) Priorität : **29.05.81 DE 3121355**
**12.06.81 DE 3123312**

(43) Veröffentlichungstag der Anmeldung : **08.12.82 Patentblatt 82/49**

(45) Bekanntmachung des Hinweises auf die Patenterteilung : **28.08.85 Patentblatt 85/35**

(84) Benannte Vertragsstaaten : **AT BE CH DE FR GB IT LI LU NL SE**

(56) Entgegenhaltungen :
**DE-A- 2 439 104**
**CHEMICAL ABSTRACTS, Band 94, 1981, Seite 94, Nr. 11639g, Columbus Ohio (USA);**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Jahn, Dieter, Dr.**
**Burgunder Weg 8**
**D-6803 Neckarhausen (DE)**
Erfinder : **Becker, Rainer, Dr.**
**Sonnenwendstrasse 83**
**D-6702 Bad Duerkheim (DE)**
Erfinder : **Goetz, Norbert, Dr.**
***Schoefferstrasse* 25**
**D-6520 Worms 1 (DE)**
Erfinder : **Wuerzer, Bruno, Dr., Dipl.-Landwirt**
**Ruedigerstrasse 13**
**D-6701 Otterstadt (DE)**

**EP 0 066 195 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Cyclohexan-1,3-dionderivate, Verfahren zu ihrer Herstellung sowie Herbizide, die diese Verbindungen als Wirkstoffe enthalten.

Es ist bekannt, daß Cyclohexan-1,3-dionderivate geeignete Herbizide zur selektiven Bekämpfung von unerwünschten Gräsern in breitblättrigen Kulturen sind (DE-AS-24 39 104).

Es wurde gefunden, daß Cyclohexan-1,3 dionderivate der Formel

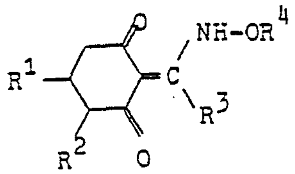

(I)

in der

$R^1$ einen fünf- oder sechsgliedrigen aromatischen, heterocyclischen Rest mit einem oder zwei Stickstoffatomen, der einen oder zwei Substituenten aus der Gruppe Alkyl mit 1 bis 2 Kohlenstoffatomen, Halogen, Alkoxi mit 1 bis 2 Kohlenstoffatomen oder Dialkylamino mit 1 bis 4 Kohlenstoffatomen in der Alkylgruppe tragen kann,

$R^2$ Wasserstoff, Methoxicarbonyl oder Ethoxicarbonyl,

$R^3$ Alkyl mit 1 bis 4 Kohlenstoffatomen und

$R^4$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogenatomen oder Propargyl bedeuten,

und Salze dieser Verbindungen eine überraschend starke und überlegene herbizide Wirkung gegen Pflanzenarten aus der Familie der Gräser (Gramineen) haben und gleichzeitig ein hohes Maß an Verträglichkeit für breitblättrige und sonstige nicht zur Familie der Gramineen zählende Kulturpflanzen besitzen.

Die Verbindungen der Formel I können in mehreren tautomeren Formen auftreten, die alle vom Patentanspruch umfaßt werden :

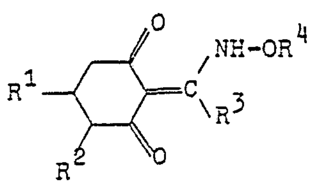

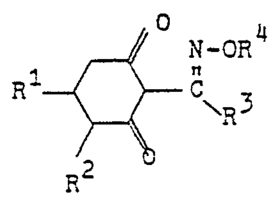

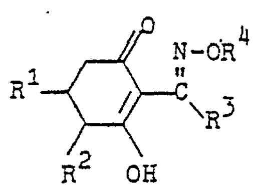

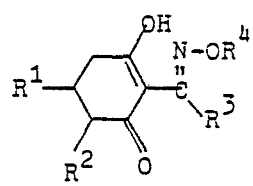

$R^1$ in Formel I bedeutet fünf- oder sechsgliedrige aromatische heterocyclische Reste, wie Pyridyl-, Pyrimidyl-, Pyrazinyl-, Pyridazinyl-, Pyrrolyl-, Imidazolyl- oder Pyrazolylreste, die jeweils einen oder zwei Substituenten tragen können. Als Substituenten kommen Halogen, wie Chlor oder Jod, Alkyl- oder Alkoxireste mit 1 oder 2 Kohlenstoffatomen, wie Methyl, Ethyl, Methoxy, Ethoxy, oder Dialkylaminoreste mit 1 bis 4 Kohlenstoffatomen in einer Alkylgruppe, wie Dimethylamino, Diethylamino, in Betracht.

Beispiele für solche Reste für $R^1$ sind Pyrid-2-yl, Pyrid-3-yl, Pyrid-4-yl, 2-Chlorpyrid-3-yl, 2-Chlorpyrid-4-yl, 2-Chlorpyrid-5-yl, 2-Chlorpyrid-6-yl, 2-Methylpyrid-6-yl, 2-Methylpyrid-5-yl, 3-Methylpyrid-4-yl, 4-Methylpyrid-3-yl, 2,6-Dimethylpyrid-4-yl, 2,4-Dimethylpyrid-6-yl, Pyrrol-2-yl, N-Methylpyrrol-2-yl, N-Methylpyrazol-2-yl, N-Methylpyrazol-4-yl.

$R^3$ in Formel I steht für unverzweigte oder verzweigte Alkylreste mit 1 bis 4 Kohlenstoffatomen, d. h. für Methyl, Ethyl, n-Propyl, i-Propyl, n-Butyl, sec.-Butyl, i-Butyl, tert.-Butyl.

Reste für $R^4$ in Formel I sind Alkyl mit 1 bis 3 Kohlenstoffatomen, Alkenyl mit 3 oder 4 Kohlenstoffatomen oder Halogenalkenyl mit 3 oder 4 Kohlenstoffatomen, das bis zu drei Halogensubstituenten trägt, beispielsweise Methyl, Ethyl, n-Propyl, i-Propyl, n-Butyl, sec.-Butyl, i-Butyl, tert.-Butyl, Allyl, 1-Chlorprop-1-en-3-yl, 2-Chlorprop-1-en-3-yl, 1,3-Dichlorprop-1-en-3-yl, 1,1,2-Trichlorprop-1-en-3-yl, oder Propargyl.

Als Salze der Verbindungen der Formel I kommen beispielsweise die Alkalimetallsalze, insbesondere die Kalium- oder Natriumsalze, die Mangan- Kupfer-, Zink-, Eisen- und Bariumsalze in Betracht.

Bevorzugt sind Verbindungen der Formel I, bei denen $R^1$ einen Pyrid-3-yl-rest, $R^2$ Wasserstoff, $R^3$ einen Alkylrest, ausgewählt aus der Gruppe Ethyl, n-Propyl, i-Propyl, und $R^4$ Ethyl oder Allyl bedeuten.

Die Verbindungen der Formel I können durch Umsetzung von Verbindungen der Formel

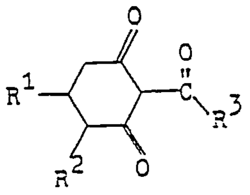

(II)

in der $R^1$, $R^2$ und $R^3$ die obengenannten Bedeutungen haben, mit Hydroxylaminderivaten $R^4O—NH_3Y$, in der $R^4$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 7, insbesondere von 4,5 bis 5,5. Die Einstellung des pH-Bereichs erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^4O—NH_3Y$, zugesetzt.

Als Lösungsmittel sind beispielsweise Dimethylsulfoxid, Alkohole, wie Methanol, Ethanol, Isopropanol, Benzol, chlorierte Kohlenwasserstoffe, wie Chloroform, Dichlorethan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^4O—NH_2$, in der $R^4$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Methylenchlorid, Toluol, Dichlorethan, Ester, wie Essigsäureethylester, Nitrile, wie Acetonitil, cyclische Ether, wie Tetrahydrofuran.

Die Alkalimetallsalze der Verbindungen der Formel I können durch Behandeln dieser Verbindungen mit Natrium- oder Kaliumhydroxid in wäßriger Lösung oder in einem organischen Lösungsmittel, wie Methanol, Ethanol, Aceton, erhalten werden. Auch Natrium- und Kaliumalkoholate können als Basen dienen.

Die anderen Metallsalze, z. B. die Mangan-, Kupfer-, Zink-, Eisen- und Bariumsalze können aus den Natriumsalzen durch Reaktion mit den entsprechenden Metallchloriden in wäßriger Lösung hergestellt werden.

Die Verbindungen der Formel II können aus Cyclohexan-1,3-dionen der Formel III, die auch in den tautomeren Formeln IIIa und IIIb vorliegen können.

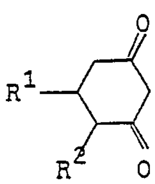

(III)

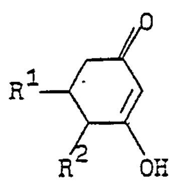

(IIIa)

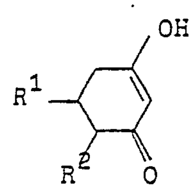

(IIIb)

nach literaturbekannten Methoden (Tetrahedron Letters, 29, 2491 (1975)) hergestellt werden.

Es ist auch möglich, Verbindungen der Formel II über die Zwischenstufe der Enolester, die bei der Umsetzung von Verbindungen der Formel III eventuell als Isomerengemische anfallen und in Gegenwart von Imidazol- oder Pyridinderivaten umgelagert werden (JP-OS 54/063052), herzustellen.

Zu den Verbindungen der Formel III gelangt man nach literaturbekannten Verfahren, wie dies aus folgendem Schema hervorgeht :

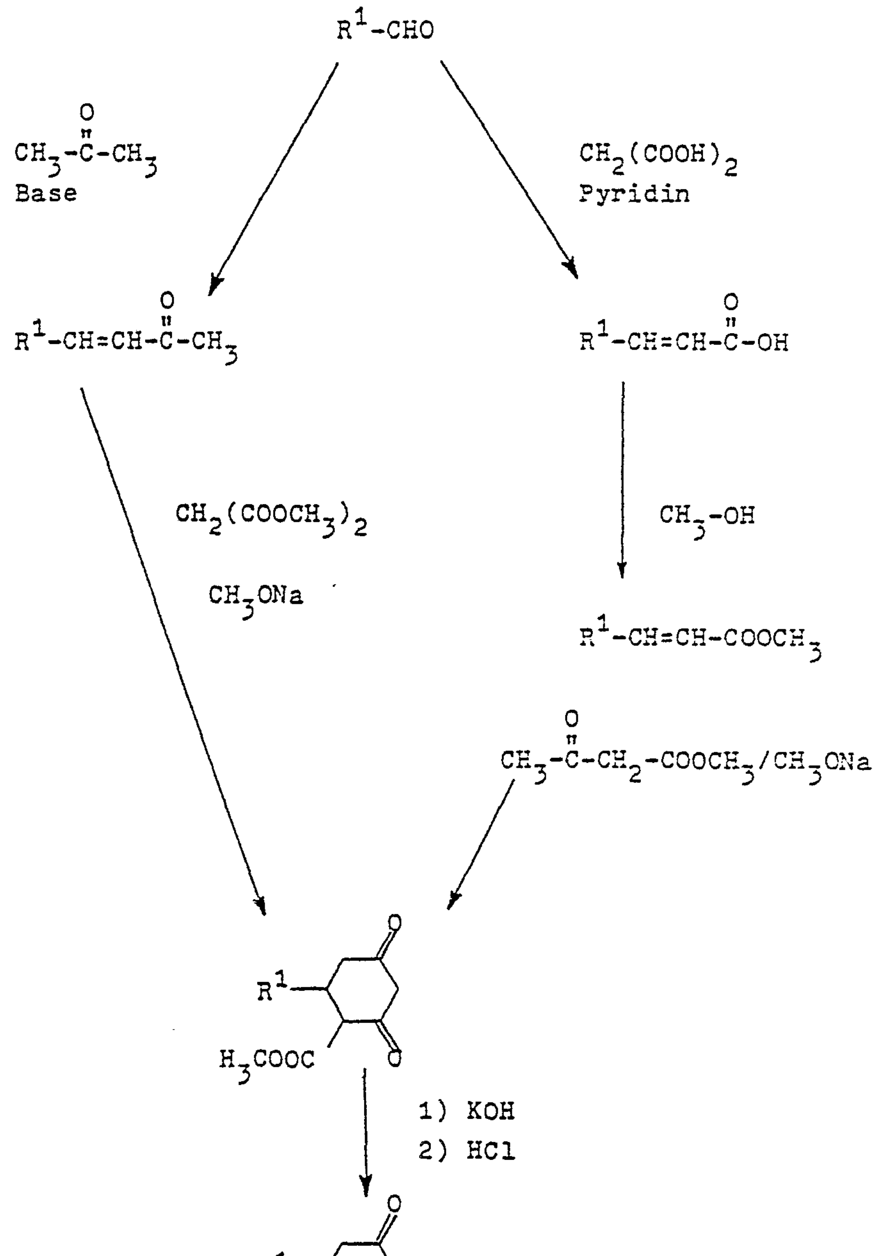


Entsprechend den folgenden Beispielen wurden Cyclohexan-1,3-dionderivate der Formel I erhalten. In den Beispielen verhalten sich Gewichtsteile zu Volumenteilen wie Kilogramm zu Liter.

Beispiel 1

3,1 Gew.-Teile Ethoxyammoniumchlorid, 7,8 Gew.Teile 2-Butyryl-5-pyrid-3-yl-cyclohexan-1,3-dion, 2,7 Gew.-Teile wasserfreies Natriumacetat und 100 Volumenteile Ethanol werden 12 Stunden bei Raumtemperatur gerührt. Das Lösungsmittel wird unter vermindertem Druck abdestilliert, der Rückstand wird mit 120 Teilen Wasser und 100 Teilen Methylenchlorid gerührt, die organische Phase abgetrennt, die wäßrige Phase mit 50 Teilen Methylenchlorid extrahiert, die vereinigten organischen Phasen mit Wasser gewaschen, über Natriumsulfat getrocknet und unter vermindertem Druck eingeengt. Man erhält ein Öl, das allmählich erstarrt. Der Feststoff wird mit Diethylether gerührt. Nach Absaugen des Rückstandes erhält man 2-Ethoxyaminobutyliden-5-(pyrid-3-yl)-cyclohexan-1,3-dion vom Fp. 80-82 °C (Ausbeute : 90 % d. Th.) (Wirkstoff Nr. 1).

$C_{17}H_{22}N_2O_3$ (302)
ber. : C 67,53 H 7,3 N 9,26
gef. : C 67,3 H 7,2 N 9,3

## Beispiel 2

2,2 Gew.-Teile Allyloxyamin, 7,8 Gew.-Teile 2-Butyryl-5-(pyrid-3-yl)-cyclohexan-1,3-dion und 100 Volumenteile Ethanol werden bei Raumtemperatur 12 Stunden gerührt. Das Lösungsmittel wird im Vakuum abdestilliert, der Rückstand in 200 Teilen Methylenchlorid aufgenommen, die Lösung zweimal mit Wasser gewaschen, über Natriumsulfat getrocknet und das Lösungsmittel im Vakuum abdestilliert. Man erhält ein Öl, das allmählich erstarrt. Der Feststoff wird mit Diethylether gerührt, der Rückstand abgesaugt. Man erhält 2-Allyloxyaminobutyliden-5-(pyrid-3-yl)-cyclohexan-1,3-dion vom Fp. 87-91 °C (Ausbeute : 94 % d. Th.) (Wirkstoff Nr. 2).

$C_{18}H_{22}N_2O_3$ (314)
ber. : C 68,77 H 7,05 N 8,91
gef. : C 68,3 H 6,9 N 9,0

Die folgenden Verbindungen werden in gleicher Weise erhalten :

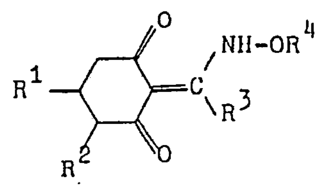

| Nr. | $R^1$ | $R^2$ | $R^3$ | $R^4$ | $n_D$/Fp [°C] |
|---|---|---|---|---|---|
| 3 | Pyrid-3-yl | H | $C_2H_5$ | $C_2H_5$ | Fp 76- 78 |
| 4 | " | H | $C_2H_5$ | $-CH_2-CH=CH_2$ | Fp 81- 83 |
| 5 | " | H | $CH_3$ | $C_2H_5$ | Fp 65- 69 |
| 6 | " | H | $CH_3$ | $-CH_2-CH=CH_2$ | Fp 87- 89 |
| 7 | " | H | $n-C_3H_7$ | $-CH_2-C\equiv CH$ | |
| 8 | " | H | $n-C_3H_7$ | $-CH_2-CH=CHCl$ | |
| 9 | " | $COOCH_3$ | $n-C_3H_7$ | $-CH_2-CH=CH_2$ | $n_D^{22}$ 1,549 |
| 10 | Pyrid-2-yl | H | $n-C_3H_7$ | $C_2H_5$ | |
| 11 | " | H | $n-C_3H_7$ | $-CH_2-CH=CH_2$ | $n_D^{22}$ 1,556 |
| 12 | 6-Methylpyrid-2-yl | H | $n-C_3H_7$ | $C_2H_5$ | $n_D^{24}$ 1,551 |
| 13 | " | H | $n-C_3H_7$ | $-CH_2-CH=CH_2$ | $n_D^{24}$ 1,556 |
| 14 | " | H | $C_2H_5$ | $C_2H_5$ | $n_D^{24}$ 1,556 |
| 15 | " | H | $C_2H_5$ | $-CH_2-CH=CH_2$ | $n_D^{24}$ 1,561 |
| 16 | Pyrrol-2-yl | H | $n-C_3H_7$ | $C_2H_5$ | |
| 17 | " | H | $n-C_3H_7$ | $-CH_2-CH=CH_2$ | |
| 18 | N-Methylpyrrol-2-yl | H | $n-C_3H_7$ | $C_2H_5$ | $n_D^{23}$ 1,556 |
| 19 | " | H | $n-C_3H_7$ | $-CH_2-CH=CH_2$ | $n_D^{23}$ 1,560 |
| 20 | N-Methylpyrazol-3-yl | H | $n-C_3H_7$ | $C_2H_5$ | |
| 21 | " | H | " | $-CH_2-CH=CH_2$ | |
| 22 | N-Methylpyrazol-4-yl | H | " | $C_2H_5$ | |
| 23 | " | H | " | $-CH_2-CH=CH_2$ | |
| 24 | 4-Methylpyrid-3-yl | H | " | $C_2H_5$ | |
| 25 | " | H | " | $-CH_2-CH=CH_2$ | |
| 26 | Pyrid-3-yl (Natriumsalz) | H | " | $C_2H_5$ | 164-168 |
| 27 | " | H | " | $-CH_2-CH=CH_2$ | 116-121 |

(Fortsetzung)

| Nr. | $R^1$ | $R^2$ | $R^3$ | $R^4$ | $n_D$/Fp [°C] |
|---|---|---|---|---|---|
| 28 | Pyrid-4-yl | H | $C_2H_5$ | $C_2H_5$ | |
| 29 | " | H | $n-C_3H_7$ | $C_2H_5$ | 80- 85 |
| 30 | " | H | " | $-CH_2-CH=CH_2$ | |
| 31 | Pyrid-3-yl (Natriumsalz) | H | $C_2H_5$ | $C_2H_5$ | 105-109 (Zers.) |
| 32 | " (Natriumsalz) | H | " | $-CH_2-CH=CH_2$ | |
| 33 | " (Kaliumsalz) | H | $n-C_3H_7$ | $C_2H_5$ | |
| 34 | " (Kupfersalz) | H | " | " | |
| 35 | " (Calciumsalz) | H | " | " | |
| 36 | " (Eisensalz) | H | " | " | |
| 37 | Pyrimid-5-yl | H | " | " | 57-60 |
| 38 | " | H | H | $-CH_2-CH=CH_2$ | $n_D^{24}$ 1,5616 |
| 39 | 2-Methoxypyrimid-5-yl | H | $n-C_3H_7$ | $-CH_2-CH=CH_2$ | |
| 40 | " | H | " | $C_2H_5$ | |
| 41 | " | H | " | $-CH_2-CH=CH_2$ | |
| 42 | 4,6-Dimethoxypyrimid-5-yl | H | $n-C_3H_7$ | $-CH_2-CH=CH_2$ | |
| 43 | " | H | " | $C_2H_5$ | |
| 44 | Pyrid-3-yl | H | " | $-CH_2-CH=CHCl$ | |
| 45 | Pyrid-2-yl | H | " | " | $n_D^{21}$: 1,568 |
| 46 | 1-Methylpyrrol-2-yl | $COOCH_3$ | " | $C_2H_5$ | Fp: 98-102 |
| 47 | " (Natriumsalz) | H | " | " | |
| 48 | " | H | $C_2H_5$ | " | |
| 49 | " | H | " | $-CH_2-CH=CH_2$ | |
| 50 | " (Natriumsalz) | H | " | " | |
| 51 | " (Natriumsalz) | H | " | $C_2H_5$ | |
| 52 | 1-Methylpyrrol-3-yl | H | " | " | |
| 53 | " | H | " | $-CH_2-CH=CH_2$ | |
| 54 | " | H | $n-C_3H_7$ | " | |
| 55 | " | H | " | $C_2H_5$ | |
| 56 | " (Natriumsalz) | H | " | " | |
| 57 | " (Natriumsalz) | H | " | $-CH_2-CH=CH_2$ | |
| 58 | 1-Methylpyrrol-3-yl (Natriumsalz) | H | $C_2H_5$ | $C_2H_5$ | |
| 59 | 1-Methylpyrrol-3-yl (Natriumsalz) | H | " | $-CH_2-CH=CH_2$ | |
| 60 | 1-Methylpyrazol-3-yl | H | " | " | |
| 61 | " | H | " | $C_2H_5$ | |
| 62 | " | $COOCH_3$ | $n-C_3H_7$ | " | $n_D^{24}$: 1,529 |
| 63 | " | " | " | $-CH_2-CH=CH_2$ | $n_D^{24}$: 1,530 |
| 64 | " | H | " | " | $n_D^{23}$: 1,546 |
| 65 | " | H | " | $C_2H_5$ | $n_D^{23}$: 1,541 |
| 66 | 1-Methylpyrazol-3-yl (Natriumsalz) | H | $n-C_3H_7$ | $C_2H_5$ | |
| 67 | 1-Methylpyrazol-3-yl (Natriumsalz) | H | " | $-CH_2-CH=CH_2$ | |

(Fortsetzung)

| Nr. | $R^1$ | $R^2$ | $R^3$ | $R^4$ | $n_D$/Fp [°C] |
|---|---|---|---|---|---|
| 68 | 1-Methylpyrazol-4-yl | H | " | " | Fp 63- 66 |
| 69 | " | H | " | $C_2H_5$ | Fp 80- 84 |
| 70 | " | H | $C_2H_5$ | " | |
| 71 | " | H | " | $-CH_2-CH=CH_2$ | |
| 72 | " | $COOCH_3$ | $n-C_3H_7$ | " | $n_D^{23}$: 1,540 |
| 73 | " | " | " | $C_2H_5$ | $n_D^{23}$: 1,535 |
| 74 | " (Natriumsalz) | H | " | " | |
| 75 | " (Natriumsalz) | H | " | $-CH_2-CH=CH_2$ | |
| 76 | Pyrimid-2-yl | H | " | " | |
| 77 | " | H | " | $C_2H_5$ | |
| 78 | " (Natriumsalz) | H | " | " | |
| 79 | Pyrimid-4-yl | H | " | " | |
| 80 | " (Natriumsalz) | H | " | " | |
| 81 | " | H | " | $-CH_2-CH=CH_2$ | |
| 82 | 2-Methylpyrimid-4-yl | H | " | " | |
| 83 | " | H | " | $C_2H_5$ | |
| 84 | 2-Methylpyrimid-4-yl (Natriumsalz) | H | $n-C_3H_7$ | $C_2H_5$ | |
| 85 | 2,6-Dimethylpyrimid-4-yl | H | " | " | |
| 86 | " (Natriumsalz) | H | " | " | |
| 87 | " | H | " | $-CH_2-CH=CH_2$ | |
| 88 | Pyridazin-3-yl | H | " | " | |
| 89 | " | H | " | $C_2H_5$ | |
| 90 | " (Natriumsalz) | H | " | " | |
| 91 | Pyridazin-4-yl | H | " | " | |
| 92 | " (Natriumsalz) | H | " | " | |
| 93 | " | H | " | $-CH_2-CH=CH_2$ | |
| 94 | 2-Methylpyrid-5-yl | H | " | $C_2H_5$ | |
| 95 | " (Natriumsalz) | H | " | " | |
| 96 | " | H | " | $-CH_2-CH=CH_2$ | |
| 97 | 4-Methylpyrid-2-yl | H | " | " | |
| 98 | " | H | " | $C_2H_5$ | |
| 99 | " (Natriumsalz) | H | " | " | |
| 100 | 3-Methoxipyrid-2-yl | H | " | " | |
| 101 | " | H | " | " | |
| 102 | 3-Methoxipyrid-2-yl | H | $n-C_3H_7$ | $-CH_2-CH=CH_2$ | |
| 103 | 2,6-Dimethoxi-4-methyl- -pyrid-3-yl | H | " | " | |
| 104 | " | H | " | $C_2H_5$ | |
| 105 | " (Natriumsalz) | H | " | " | |

Die erfindungsgemäßen Substanzen können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsufloxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 10 Gewichtsteile des Wirkstoffs Nr. 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile des Wirkstoffs Nr. 10 werden in einer Mischung gelöst, die aus 60 Gewichsteilen Cyclohexanon, 30 Gewichtsteilen Isobutanol, 5 Gewichtsteilen des Anlagerungsproduktes von 7 Mol Ethylenoxid an 1 Mol Isooctyphenol und 5 Gewichtsteilen des Anlagerungsproduktes von 40 Mol Ethylenoxid an 1 Mol Ricinusöl besteht.

IV. 20 Gewichtsteile des Wirkstoffs Nr. 2 werden in einer Mischung gelöst, die aus 25 Gewichtsteilen Cyclohexanol, 65 Gewichtsteilen einer Mineralölfraktion vom Siedepunkt 210 bis 280 °C und 10 Gewichsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile des Wirkstoffs Nr. 2 werden mit 3 Gewichtsteilen des Natriumsalzes der Diisobutylnaphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile des Wirkstoffs Nr. 1 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile des Wirkstoffs Nr. 11 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 10 werden mit 2 Teilen Calciumsalz der Dodecylbenzolsulfonsäure, 8

Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflaufverfahren, während des Auflaufens der unerwünschten Pflanzen oder nach deren Auflaufen erfolgen.

Die Aufwandmengen an Wirkstoff betragen je nach Anwendungsmethode, Art und Wachstummstadium der zu bekämpfenden Pflanzen 0,025 bis 10 kg/ha und mehr. Vorzugsweise liegt die Aufwandmenge zwischen 0,1 und 1,5 kg/ha.

Sind gewisse Kulturpflanzen gegenüber den Wirkstoffen weniger tolerant, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by). In Anbetracht der guten Verträglichkeit und der Vielseitigkeit der Applikationsmethoden können die Wirkstoffe der Formel I oder diese enthaltende herbizide Mittel in einer großen Zahl von Kulturpflanzen zur Beseitigung unerwünschten Pflanzenwuchses eingesetzt werden.

In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotania tabacum (N. rustica) | Tabak |
| Olea europaea | Ölbaum |
| Phaseolus lunatus | Mondbohne |
| Phaseolus mungo | Erdbohne |

| | |
|---|---|
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Sasamum indicum | Sesam |
| Solanum tuberosum | Kartoffel |
| Spinacia oleracea | Spinat |
| Theobroma cacao | Kakaobaum |
| Trifolium pratense | Rotklee |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays (große Pflanzen, Unterblattspritzung (post-directed)) | Mais |

Die Wirkung der Cyclohexan-1,3-dionderivate der Formel I auf das Wachstum von Pflanzen aus der Gräserfamilie (Gramineen) und breitblättrigen Kulturpflanzen läßt sich durch Gewächshausversuche zeigen. Dabei können auch Kulturpflanzen aus der Familie der Gramineen absterben oder stark geschädigt werden. Dies kann in der Praxis durchaus erwünscht sein, da auch Kulturpflanzen zu unerwünschten Pflanzen werden können, wenn sie aus im Boden zurückgebliebenem Samen in einer anderen Kultur aufwachsen, wie z. B. Ausfallgerste (voluntary barley) in Winterraps oder Sorghum in Sojabohnenfeldern.

Als Kulturgefäße für die Versuche dienten Plastikblumentöpfe mit 300 $cm^3$ Inhalt und lehmigem Sand mit etwa 1,5 % Humus als Substrat. Bei Soja wurde etwas Torf (peat) zugemischt, um ein besseres Wachstum zu gewährleisten. Die Samen der Testpflanzen wurden nach Arten getrennt flach eingesät.

Bei der Vorauflaufbehandlung wurden die Wirkstoffe auf die Erdoberfläche aufgebracht. Sie wurden hierzu in Wasser als Verteilungsmitel suspendiert oder emulgiert und mittels fein verteilender Düsen gespritzt. Bei dieser Applikationsmethode betrug die Aufwandmenge 0,125 bzw. 3,0 kg Wirkstoff/ha. Nach dem Aufbringen der Mittel wurden die Gefäße leicht beregnet, um Keimung und Wachstum in Gang zu bringen. Danach deckte man die Gefäßte mit durchsichtigen Plastikhauben ab, bis die Pflanzen angewachsen waren. Die Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Testpflanzen je nach Wuchsform erst bis zu einer Wuchshöhe von 3 bis 10 cm an und behandelte sie danach. Die Aufwandmenge für die Nachauflaufbehandlung betrug 0,125, 0,25 bzw. 0,5 kg Wirkstoff/ha.

Die Versuchsperiode erstreckte sich über 2 bis 4 Wochen. Während dieser Zeit wurden die Pflanzen gepflegt, und ihre Reaktion auf die einzelnen Behandlungen wurde ausgewertet. Bewertet wird nach einer Skala von 0 bis 100. Dabei bedeutet 0 keine Schädigung oder normaler Aufgang und 100 kein Aufgang bzw. völlige Zerstörung zumindest der oberirdischen Sproßteile.

Getestet wurden die folgenden Pflanzen :

Alopecurus myosuroides (Acker-Fuchsschwanz), Avena fatua (Flughafer), Avena sativa (Hafer), Beta vulgaris (Zuckerrübe), Brassica napus (Raps), Bromus tectorum (Dach-Trespe), Bromus spp. (Trespearten), Echinochloa crusgalli (Hühnerhirse), Gossypium hirsutum (Baumwolle), Glycine max (Soja), Hordeum vulgare (Gerste), Lolium multiflorum (Ital. Raygras), Rottboellia exaltata, Sorghum bicolor (Mohrenhirse, Kulturhirse), Sorghum halepense (Aleppohirse), Setaria spp. (Borstenhirsearten), Triticum aestivum (Weizen).

In den Versuchen zeigten die Cyclohexan-1,3-dionderivate der Formel I eine sehr starke Wirkung gegen Pflanzenarten aus der Familie der Gräser (Gramineen), während die Wirkstoffe in hohem Maße für breitblättrige Kulturpflanzen verträglich waren. Beispielsweise zeigten die Verbindungen Nr. 3, 4, 5, 26, 27, 29, 1 und 2 bei der Prüfung auf herbizide Wirksamkeit bei Nachauflaufanwendung mit 0,125 bzw. 0,25 kg/ha Wirkstoff eine sehr starke herbizide Wirkung gegen Gramineen, ohne die Kulturpflanze Soja zu schädigen. Durch die Verbindungen Nr. 12 und 13 wurden nicht nur grasartige Pflanzen in breitblättrigen Kulturen selektiv bekämpft, die Verbindungen waren auch für Weizen selektiv.

Bei Vorauflaufanwendung von 3,0 kg/ha Wirkstoff zeigten die Wirkstoffe Nr. 1, 2, 10 und 11 eine beachtliche Aktivität gegen Pflanzenarten aus der Gräserfamilie.

Zur Verbreiterung des Wirkungsspektrums und zur Erzielung synergistischer Effekte können die Cyclohexan-1,3-dionderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazin-derivate, Benzothiadiazinone, 2,6-Dinitroaniline, N-Phenylcarbamate, Thiocarbamate, Halogencarbonsäuren, Triazine, Amide, Harnstoffe, Diphenylether, Triazinone, Uracile, Benzofuranderivate und andere in Betracht.

Außerdem ist es nützlich, die erfindungsgemäßen Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können auch Netz- und Haftmittel sowie nicht-phytotoxische Öle und Ölkonzentrate zugesetzt werden.

**Patentansprüche**

1. Cyclohexan-1,3-dionderivate der Formel

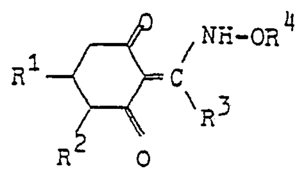

(I)

in der

$R^1$ einen fünf- oder sechsgliedrigen aromatischen, heterocyclischen Rest mit einem oder zwei Stickstoffatomen, der einen oder zwei Substituenten aus der Gruppe Alkyl mit 1 bis 2 Kohlenstoffatomen, Halogen, Alkoxi mit 1 bis 2 Kohlenstoffatomen oder Dialkylamino mit 1-4 Kohlenstoffatomen in einer Alkylgruppe, tragen kann,

$R^2$ Wasserstoff, Methoxicarbonyl oder Ethoxicarbonyl,

$R^3$ Alkyl mit 1 bis 4 Kohlenstoffatomen und

$R^4$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogenatomen oder Propargyl bedeuten,

und Salze dieser Verbindungen.

2. Cyclohexan-1,3-dionderivate der Formel I gemäß Anspruch 1, dadurch gekennzeichnet, daß $R^1$ einen Pyrid-3-yl-Rest, $R^2$ Wasserstoff, $R^3$ einen Alkylrest ausgewählt aus der Gruppe Ethyl, n-Propyl, i-Propyl und $R^4$ Ethyl oder Allyl bedeuten.

3. 2-Ethoxiaminobutyliden-5-(pyrid-3-yl)-cyclohexan-1,3-dion.

4. 2-Allyloxiaminobutylien-5-(pyrid-3-yl)-cyclohexan-1,3-dion.

5. Verfahren zur Herstellung eines Cyclohexan-1,3-dionderivates der Formel I gemäß Anspruch 1, dadurch gekennzeichnet, daß man eine Verbindung der Formel

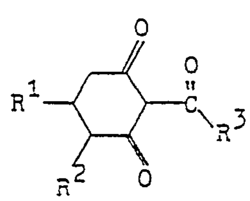

(II)

in der $R^1$, $R^2$ und $R^3$ die im Anspruch 1 genannten Bedeutungen haben,

a) mit einer Ammoniumverbindung der Formel $R^4O—NH_3Y$, in der $R^4$ die im Anspruch 1 genannten Bedeutungen hat und Y ein Anion bedeutet, in einem inerten Verdünnungsmittel bei einer Temperatur zwischen 0 und 80 °C in Gegenwart einer Base oder

b) mit einem Hydroxylamin der Formel $R^4O—NH_2$, in der $R^4$ die im Anspruch 1 genannten Bedeutungen hat, in einem inerten Lösungsmitel umsetzt,

6. Verfahren nach Anspruch 5, dadurch gekennzeichnet, daß man eine Verbindung der Formel II mit einer Ammoniumverbindung der Formel $R^4O—NH_3Y$ bei einem pH-Wert im Bereich zwischen 2 und 7 umsetzt.

7. Herbizid, enthaltend inerte Zusatzstoffe und ein Cyclohexan-1,3-dionderivat der Formel

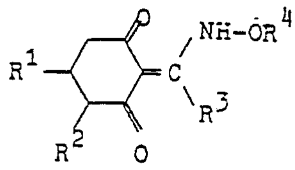

(I)

in der

$R^1$ einen fünf- oder sechsgliedrigen aromatischen, heterocyclischen Rest mit einem oder zwei Stickstoffatomen, der einen oder zwei Substituenten aus der Gruppe Alkyl, Halogen, Alkoxi oder Dialkylamino tragen kann,

$R^2$ Wasserstoff, Methoxicarbonyl oder Ethoxicarbonyl,

$R^3$ Alkyl mit 1 bis 4 Kohlenstoffatomen und

$R^4$ Alkyl mit 1 bis 3 Kohlenstoffatomen, Alkenyl mit 3 oder 4 Kohlenstoffatomen, Halogenalkenyl mit 3 oder 4 Kohlenstoffatomen und 1 bis 3 Halogenatomen oder Propargyl bedeuten, oder Salze dieser Verbindungen.

8. Herbizid, enthaltend inerte Zusatzstoffe und ein Cyclohexan-1,3-dionderivat der Formel I gemäß Anspruch 1, wobei $R^1$ einen Pyrid-3-yl-Rest, $R^2$ Wasserstoff, $R^3$ einen Alkylrest ausgewählt aus der Gruppe Ethyl, n-Propyl, i-Propyl und $R^4$ Ethyl oder Allyl bedeuten.

9. Herbizid, enthaltend inerte Zusatzstoffe und 2-Ethoxiaminobutyliden-5-(pyrid-3-yl)-cyclohexan-1,3-dion als Wirkstoff.

10. Verfahren zur Bekämpfung unerwünschten Pflanzenwachstums, dadurch gekennzeichnet, daß man die unerwünschten Pflanzen oder die von unerwünschtem Pflanzenwachstum freizuhaltende Fläche mit einer herbizid wirksamen Menge eines Cyclohexan-1,3-dion-derivates der Formel I gemäß Anspruch 1 behandelt.

**Claims**

1. A cyclohexane-1,3-dione derivative of the formula

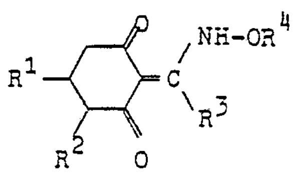

(I)

where

$R^1$ is a five-membered or six-membered aromatic heterocyclic radical which contains one or two nitrogen atoms and may bear one or two substituents from the group comprising alkyl of 1 or 2 carbon atoms, halogen, alkoxy of 1 or 2 carbon atoms and dialkylamino, where one alkyl group is of 1 to 4 carbon atoms,

$R^2$ is hydrogen, methoxycarbonyl or ethoxycarbonyl,

$R^3$ is alkyl of 1 to 4 carbon atoms, and

$R^4$ is alkyl of 1 to 3 carbon atoms, alkenyl of 3 or 4 carbon atoms, haloalkenyl of 3 or 4 carbon atoms and 1 to 3 halogen atoms, or propargyl, or a salt thereof.

2. A cyclohexane-1,3-dione derivative of the formula I as claimed in claim 1, where $R^1$ is pyrid-3-yl, $R^2$ is hydrogen, $R^3$ is alkyl selected from the group comprising ethyl, n-propyl and isopropyl, and $R^4$ is ethyl or allyl.

3. 2-Ethoxyaminobutylidine-5-(pyrid-3-yl)-cyclohexane-1,3-dione.

4. 2-Allyloxyaminobutylidene-5-(pyrid-3-yl)-cyclohexane-1,3-dione.

5. A process for the preparation of a cyclohexane-1,3-dione derivative of the formula I as claimed in claim 1, wherein a compound of the formula

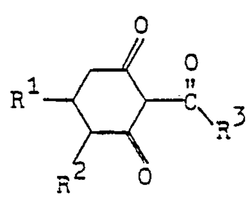

(II)

where $R^1$, $R^2$ and $R^3$ have the meanings given in claim 1, is reacted

a) with an ammonium compound of the formula $R^4O—NH_3Y$, where $R^4$ has the meanings given in claim 1 and Y is an anion, in an inert diluent, at a temperature of from 0° to 80 °C and in the presence of a base, or

b) with a hydroxylamine of the formula $R^4O—NH_2$, where $R^4$ has the meanings given in claim 1, in an inert solvent.

6. A process as claimed in claim 5, wherein a compound of the formula II is reacted with an ammonium compound of the formula $R^4O—NH_3Y$ at a pH of from 2 to 7.

7. A herbicide containing inert additives and a cyclohexane-1,3-dione derivative of the formula I as claimed in claim 1.

8. A herbicide containing inert additives and a cyclohexane-1,3-dione derivative of the formula I as claimed in claim 1, where $R^1$ is pyrid-3-yl, $R^2$ is hydrogen, $R^3$ is alkyl selected from the group comprising ethyl, n-propyl and isopropyl, and $R^4$ is ethyl or allyl.

9. A herbicide containing inert additives and 2-ethoxyamino-butylidine-5-(pyrid-3-yl)-cyclohexane-1,3-dione as active ingredient.

10. A process for controlling unwanted plant growth, wherein the unwanted plants or the area to be kept free from unwanted plant growth are treated with a herbicidally effective amount of a cyclohexane-1,3-dione derivative of the formula I as claimed in claim 1.

**Revendications**

1. Dérivés de cyclohexane-1,3-dione de formule

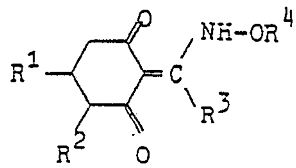

(I)

dans laquelle

$R^1$ représente un reste hétérocyclique aromatique, à cinq ou six termes, et un ou deux atomes d'azote, qui peut porter un ou deux substituants du groupe des alkyle à 1 ou 2 atomes de carbone, halogène, alcoxy à 1 ou 2 atomes de carbone ou dialkylamino à 1-4 atomes de carbone dans son groupe alkyle.

$R^2$ représente hydrogène, méthoxycarbonyle ou éthoxycarbonyle,

$R^3$ alkyle de 1 à 4 atomes de carbone et

$R^4$ alkyle de 1 à 3 atomes de carbone, alcényle à 3 ou 4 atomes de carbone, halogénalcényle à 3 ou 4 atomes de carbone et 1 à 3 atomes d'halogène ou propargyle

et sels de ces composés.

2. Dérivés de cyclohexane-1,3-dione de formule I selon la revendication 1, caractérisés par le fait que $R^1$ représente un reste pyrid-3-yle, $R^2$, hydrogène, $R^3$ un reste alkyle choisi dans le groupe éthyle, n-propyle, i-propyle et $R^4$ éthyle ou allyle.

3. 2-étyhoxyaminobutylidèn-5-(pyrid-3-yl)-cyclohexane-1,3-dione.

4. 2-allyloxyaminobutylidèn-5-(pyrid-3-yl)-cyclohexane-1,3-dione.

5. Procédé de préparation d'un dérivé de cyclohexane-1,3-dione de formule I selon la revendication 1, caractérisé par le fait qu'on fait réagir un composé de formule

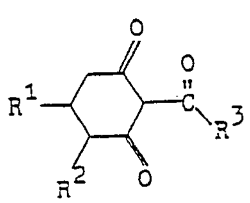

(II)

dans laquelle $R^1$, $R^2$ et $R^3$ ont les significations indiquées dans la revendication 1,

a) avec un composé d'ammonium de formule $R^4O—NH_3Y$, dans laquelle $R^4$ a les significations indiquées dans la revendication 1 et Y représente un anion, dans un diluant inerte, à une température comprise entre 0 et 80 °C, en présence d'une base ou

b) avec une hydroxylamine de formule $R^4O—NH_2$, dans laquelle $R^4$ a les significations indiquées dans la revendication 1, dans un solvant inerte.

6. Procédé selon la revendication 5, caractérisé par le fait que l'on fait réagir un composé de formule II avec un composé d'ammonium de formule $R^4O—NH_3Y$, à un pH compris entre 2 et 7.

7. Herbicide, contenant des additifs inertes et un dérivé de cyclohexane-1,3-dione de formule I selon la revendication 1.

8. Herbicide, contenant des additifs inertes et un dérivé de cyclohexane-1,3-dione de formule I selon

la revendication 1, $R^1$ représentant un reste pyrid-3-yle, $R^2$, hydrogène, $R^3$ un reste alkyle choisi dans le groupe éthyle, n-propyle, i-propyle et $R^4$ éthyle ou allyle.

9. Herbicide, contenant des additifs inertes et, comme principe actif, de la 2-éthoxyaminobutylidèn-5-(pyrid-3-yl)-cyclohexane-1,3-dione.

10. Procédé de lutte contre la croissance indésirable de plantes, caractérisé par le fait que l'on traite les plantes indésirables, ou les surfaces à maintenir libres de la croissance indésirable des plantes, avec une quantité efficace au point de vue herbicide d'un dérivé de cylohexane-1,3-dione de formule I selon la revendication 1.